# EUROPEAN PATENT APPLICATION

(11) **EP 0 769 827 A1**
(43) Date of publication of application: **23.04.1997**
(21) Application number: 96850168.4
(22) Date of filing: 11.10.1996
(51) Int. Cl.: H01R 4/48, A61N 1/375

(54) **End connector fixation plug**

(30) Priority: 18.10.1995 SE 9503646
(71) Applicant: Pacesetter AB, 171 95 Solna (SE)
(72) Inventor: Fröberg, Paul, 161 70 Bromma (SE)
(74) Representative: Winblad, Hans Peter

(57) **Abstract**

The invention relates to a plug (10) for attachment to and establishing contact with an end connector (30) on an electrical conductor, such as an electrode cable, for a medical device. The plug consists of two separate contact means (12) with at least a pair of coaxially aligned holes (18) whose cross-section is adapted to the cross-section of the end connector (30). A torsional means (20) is located between the contact means (12) and has a lead-in (24) for the end connector (30).

## Description

### Field of the invention

The present invention relates generally to plugs for attachment to and establishing contact with an end connector on an electrical conductor and relates more specifically to a plug for attachment to and establishing contact with a contact pin on the proximal end of an electrode cable in a medical device, e.g. an implantable heart stimulator such as a pacemaker. A plug connector of this kind is primarily intended for mounting in the socket section of the heart stimulator in order to achieve reliable attachment to and contact with the proximal end there.

### The prior art

Many different types of plugs have previously been proposed for plugs for establishing contact with and attachment to the proximal end of an electrode cable in a heart stimulator. The arguably most common principle in this context is to affix the proximal contact pin by inserting it into a contact socket and then connecting the pin by clamping same with a fixation screw. For example, US-A-4 072 154 shows such a fixation plug. The fixation screws used here are tiny and can easily be damaged or broken off if screwed down too tightly.

EP-A1-0 356 721 and EP-A2-0 590 756, for example, show a fixation plug for electrode contact pins, the fixation screws being replaced by spring-loaded clamping means which can be actuated by pressure exerted from outside the connector plug.

### Summary of the invention

One object of the present invention is to propose a fixation and contact plug making it possible, with a minimum number of components, to achieve simple and reliable attachment to and contact with an electrode contact pin by the use of a predefined clamping or shearing force.

For this purpose, the plug cited above is characterized by two spaced apart contact means, with oppositely located apertures, whose cross-section is adapted to the cross-section of the end connector, and a torsional means, located between the contact means, with a through passage for the end connector, the torsional means being rotatable from a passive position, in which the through passage is not aligned with the apertures in the contact means, and an active, pre-tensioned position in which the through passage is largely aligned with the apertures in the contact means. When an electrical contact pin is connected, the torsional means is turned, e.g. with a screwdriver or wrench, to the pre-tensioned position producing alignment of the through passage and the apertures. The contact pin is then inserted and manual turning terminates, the pre-tensioned torsional element, as a result of its attempt to resume its passive position, thereby clamping the pin in the apertures and exerting a shearing force on the pin. The pin is easily disconnected when the torsional means is again turned until the through passage and the apertures in the contact means are again aligned.

Additional distinguishing features of the present invention will be provided in the subsequent detailed description and in the subsequent patent claims, referring to the attached drawing.

### Description of drawings

FIG. 1 is a vertical sectional view of a fixation and contact plug, according to the present invention, in an unimpeded, inactive state;
FIG. 2 is a lateral view of the plug in FIG. 1;
FIG. 3 is a sectional view along the line III-III in FIG. 1;
FIG. 4 is a cross-section, like the one in FIG. 3, but it shows the plug in an active state for affixing and establishing contact with the contact pin inserted into the plug.

### The preferred embodiment

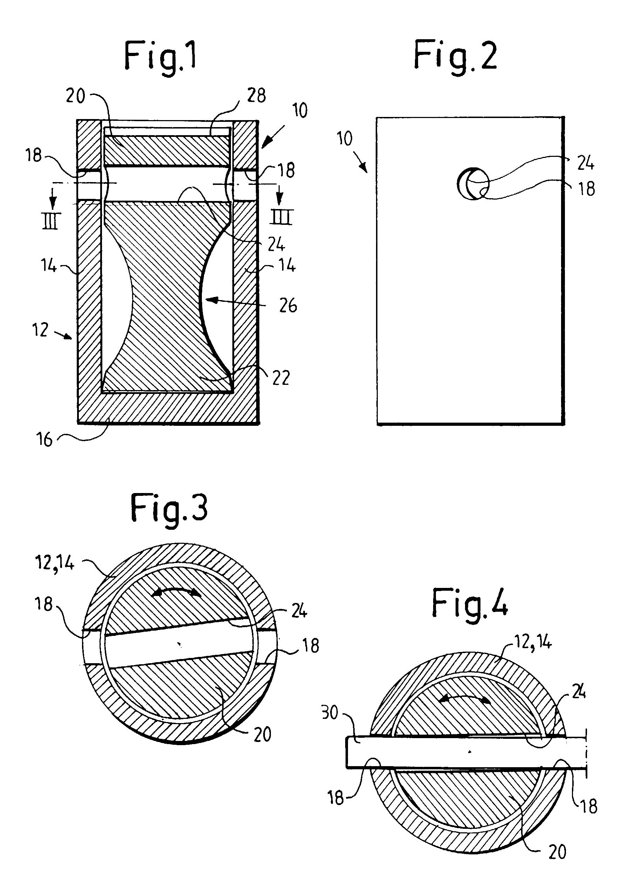

FIG. 1 shows a vertical, sectional view of a fixation and contact plug, generally designated 10, according to a preferred embodiment of the invention but not restricted to same. The fixation plug 10 contains a cylindrical, hollow contact shell 12 made of an electrically conductive material with a surrounding wall 14 and a bottom wall 16. The cylinder wall has two diametrically opposed apertures or holes 18 to admit a tightly fitting contact pin on the proximal end of the electrode cable which is to be connected e.g. to a pacemaker. A torsional means 20, whose base end is permanently attached to the bottom wall 16 of the shell 12 and whose upper section has a lead-in or through passage in the form of a hole 24 for the contact pin, is arranged inside the shell 12.

In its unimpeded, passive position depicted in FIGS. 1-3, the passage 24 is on a level with but turned out of alignment with the holes 18 in the cylinder wall 14. The torsional means 20 has a narrow "waist" 26 to facilitate rotation of the torsional means 20 on its longitudinal axis. This rotation can be achieved with e.g. a screw-turning tool, such as a screwdriver, hex wrench or the like, which is inserted into a recess or groove 28 in the upper, free end of the torsional means 20 before the contact pin is affixed, whereupon the means 20 is turned clockwise, as shown in FIG. 3, until the passage 24 aligns with the holes 18 in the shell 12. The torsional means 20 is then in a tensioned rotation position.

As shown in FIG. 4, a contact pin 30 on the end of an electrode cable (not shown) is then inserted through the holes 12 and the passage 24. When the pin 30 reaches its fixation position, the screw-turning tool is withdrawn. The torsional element will then strive to turn back to its original position, as shown in FIG. 3, clamping the pin 30 in position in the holes 18 by exerting a transverse shearing force on the pin in the area between the holes 18 and the torsional means 20. This holds the pin 30 in the holes 18 with a desired, adequate force which achieves both good electrical contact between the pin 30 and the contact shell 12 and good resistance against axial withdrawal.

A fixation socket according to the invention is mainly, but not exclusively, intended for mounting in the connection part of a pacemaker in order to conduct electrical signals between an electrode cable connected to the heart and an electronics unit in the pacemaker.

Thus, in the depicted embodiment of the plug according to the invention, fixation and contact are achieved between two axially separate sites on the contact pin 30 by means of the holes 18 in the wall of the cylindrical shell 12 with the torsional means 20 in between. However, it is possible, within the scope of the invention, for the wall sections of the contact means with contact holes to consist of spaced apart completely separate elements.

## Claims

1. A plug for fixation to and contact with an end connector (30) on an electrical conductor, such as an electrode contact pin, in a medical device, **characterized** by two spaced apart contact means (12) with oppositely located apertures (18), whose cross-section is adapted to the cross-section of the end connector (30), and a torsional means (20), located between the contact means (12), with a through passage (24) for the end connector (30), the torsional means (20) being rotatable between a passive position, in which the through passage (24) is not aligned with the apertures (18) in the contact means (12), and an active, pre-tensioned position, in which the through passage (24) is largely aligned with the apertures (18) in the contact means (12).

2. A plug according to claim 1, **characterized** in that one end section (22) of the torsional element (20) is securely mounted.

3. A plug according to claims 1 or 2, **characterized** in that the torsional means (20) has a narrow "waist" (26).

4. A plug according to claims 1-3, **characterized** in that the contact means is formed by the walls (14) of a hollow, cylindrical means (12).

5. A plug according to claim 4, **characterized** in that the hollow cylindrical means (12) has a bottom wall (16) to which the said one end (22) of the torsional means (20) is securely mounted.

6. A plug according to claim 5, **characterized** in that the torsional means is in the shape of a cylindrical rod (20), whose section containing the through passage (24) has an external diameter which is virtually the same as the internal diameter of the cylindrical wall section (14).

7. A plug according to any of claims 2-6, **characterized** in that the other end of the torsional means (20) has a means (28) for rotating the torsional means (20).

8. A plug according to claim 7, **characterized** in that the means (28) consists of a recess which fits a screw-rotation tool.
